# EUROPÄISCHE PATENTANMELDUNG

(11) **EP 1 354 958 A1**
(43) Veröffentlichungstag der Anmeldung: **22.10.2003**
(21) Anmeldenummer: 02008686.4
(22) Anmeldetag: 18.04.2002
(51) Int. Cl.: C12N 15/88, A61K 48/00

(54) **Herstellung und Anwendung von DNA-Polyelektrolyt-Nanopartikeln für den Gentransfer**

(71) Anmelder: MAX-DELBRÜCK-CENTRUM FÜR MOLEKULARE MEDIZIN, 13125 Berlin (DE); Max-Planck-Gesellschaft zur Förderung der Wissenschaften e.V., 80539 München (DE)
(72) Erfinder: Böttger, Michael, Dr., 13189 Berlin (DE); Zaitsev, Sergei, 13125 Berlin (DE); Cartier, Regis, 13125 Berlin (DE); Haberland, Annekathrin, Dr., 10430 Berlin (DE); Sukhorukov, Gleb, Dr., 14471 Potsdam (DE); Zastrow, Heidi, 14482 Postdam (DE); Schneider, Marc, 10629 Berlin (DE); Möhwald, Helmut, Prof. Dr., 55411 Bingen (DE)
(74) Vertreter: Ziebig, Marlene, Dr. Dipl.-Chem.

(57) **Zusammenfassung**

Die Erfindung betrifft ein nichtvirales Verfahren zur Transfektion von Zellkulturen, Gewebe- bzw. Organkulturen und ist unter Einhaltung bestimmter Rahmenbedingungen auch für den Gentransfer in vivo einsetzbar. Sie ist für die medizinische und biologische Grundlagenforschung, besonders im Hinblick auf Vorstudien zur Gentherapie und in der pharmazeutischen Industrie geeignet.

Das Verfahren zum Gentransfer in tierische Zellen ist dadurch gekennzeichnet, daß Vektor-DNA in wäßriger Lösung durch eine aufeinanderfolgende Addition entgegengesetzt geladener Polyelektrolyte in kompakte Nanopartikel verpackt und nachfolgend die Transfektion der Zellen durchgeführt wird.

Es ist ferner dadurch gekennzeichnet, daß die Vektor-DNA
- im ersten Schritt mit Polykationen in Wechselwirkung gebracht und kondensiert wird,
- im zweiten Schritt mit Polyanionen gemischt und eine Schicht aufgetragen wird
- und diese Schritte ggf. wiederholt werden.

Für den in vivo Gentransfer wird die Vektor-DNA in Polyelektrolytnanopartikel (Shells) verpackt, diese werden mit z. B. Pluronic-Gel F127 gemischt und in flüssiger Form auf die zu transfizierenden Gewebe/Organe/Tumoren nach deren Freilegung aufgetragen.

## Beschreibung

Die Erfindung betrifft ein nichtvirales Verfahren zur Transfektion von Zellkulturen, Gewebe- bzw. Organkulturen und ist unter Einhaltung bestimmter Rahmenbedingungen auch für den Gentransfer in vivo einsetzbar. Sie ist für die medizinische und biologische Grundlagenforschung, besonders im Hinblick auf Studien zur Gentherapie und in der pharmazeutischen Industrie geeignet.

Alle Verfahren des nichtviralen Gentransfers beruhen auf der Wechselwirkung der Transgen-DNA mit geeigneten Trägermolekülen, die im Transfektionsprozess mehrfache Funktionen übernehmen: Überführung der DNA in einen kondensierten Konformationsszustand, der die Aufnahme durch die Rezipientenzelle ermöglicht und die DNA vor einem nucleolytischem Abbau schützt. Es ist weiterhin erforderlich, lysosomolytische bzw. membranolytische Wirkungen zu integrieren, um ein Verlassen der Endosomen/Lysosomen zu ermöglichen und ein den Zellkern lokalisierendes Signal einzubauen. In der Literatur beschriebene bzw. kommerzielle Transfektantien erfüllen diese Forderungen nur teilweise. Es sind 3 Gruppen derartiger Transfektantien bekannt, zu denen z.B. die kationischen Liposomen, Peptid- bzw. Protein-vermittelte Systeme und die Polymervermittelten Systeme gehören. Diese Methoden sind weitgehend in einer aktuellen Übersicht (R.I. Mahato, L.C. Smith, A. Rolland (1999) Advances in Genet. 41, 95-156) dokumentiert und können hier nicht weiter beschrieben werden.

Ein Hauptproblem bei der Herstellung transfektionsaktiver DNA-Komplexe auf der Basis dieser Systeme ist ihre schlechte Löslichkeit in physiologischen Salzlösungen. Hierbei handelt es sich um eine von der DNA geprägte Eigenschaft, die bei ladungsneutralisierter DNA auftritt (z.B. Chromatin). Die resultierenden Aggregate schließen wegen Emboliegefahr und ungenügender Bioverteilung ihre Anwendung in der Gentherapie aus. Andererseits wurde eine direkte Korrelation zwischen Aggregatgröße und in vitro Transfektionseffizienz an praktisch allen Systemen nachgewiesen. Allerdings erwiesen sich die großen Aggregate oft als toxisch für die Zellen. Lösliche Komplexe, die neben den Aggregaten beobachtet werden, sind transfektionsinaktiv. Eine einfache Zentrifugation der Komplexe bei 4000 x g und 2 min belegt diese Befunde. Eine Ausnahme bilden Polyethylenimin- und Dendrimerkomplexe, die bei hohem positiven Ladungsüberschuß weniger zur Aggregation neigen und transfektionsaktiv sind.

Ein Genvektor für die Gentherapie sollte klein (< 100 nm) sein, um durch Endozytose aufgenommen werden zu können. Rezeptorspezifische Liganden sollten anwesend sein, um ein Zell-bzw. Organtargeting zu ermöglichen. Weiterhin sollten Genvektoren stabil sein, d.h. nicht zur Aggregation tendieren und nicht mit Serumproteinen wechselwirken. Eine hohe Transfektionsaktivität wird erwartet. Von der Realisierung dieser Eigenschaften ist die Fachwelt momentan noch weit entfernt. Bereits die Forderung nach hoher in vitro Effizienz bei geringer Größe der transfizierenden Komplexe konnte bisher noch nicht befriedigend erfüllt werden.

Ein Weg, der in der Literatur beschritten wird, um die Aggregationsneigung zu umgehen, ist die Umhüllung der transfizierenden Komplexe mit einem neutralen Polymer, als das u.a. Polyethylenglycol (PEG) herangezogen wird, und das kovalent an den DNA-Träger angekoppelt wird (M. Ogris, S. Brunner, S. Schüller, R. Kircheis, E. Wagner (1999) Gene Ther. 6, 595-605). Trubetskoy et al. haben Nanopartikel (Shells) auf der Basis von Plasmid-DNA und Polylysin hergestellt und diese Partikel mit einer Hülle aus sukzinylierten Polylysin umgeben (V.S. Trubetskoy, A. Loomis, J.E. Hagstrom, V.G. Budker, J.A. Wolff (1999) Nucleic Acids Res. 27, 3090-3095). In hypotonen Lösungen waren diese Partikel < 100 nm und zeigten nur dann eine Aggregationstendenz, wenn Polykation und Polyanion (hier Polylysin und sukzinyliertes Polylysin) im Verhältnis 1: 1 anwesend waren. Trubetskoy et al. konnten auch zeigen, daß sich nicht alle negativen Polyelektrolyte eignen, die Energie für die zur Shell-Bildung erforderlichen Ladungsumverteilungen bereitzustellen, ohne die DNA freizusetzen. Die Carboxyl/Backbone-Distanz bzw. die Ladungsdichte des Polyanions ist hierfür ein wichtiger Parameter. Kurze Distanz bzw. große Ladungsdichte führt zur Dissoziation des binären Komplexes zwischen DNA und Polylysin, große Distanz zu stabilen ternären Komplexen. Transfektionsdaten wurden nicht publiziert.

Der Erfindung lag die Aufgabe zugrunde, erste Schritte bei der Erfüllung der o.a. Forderungen durch die Anwendung eines neuen Prinzips zur Partikelassemblierung zu gehen. Insbesondere sollten kleine, stabile Partikel entwickelt werden, die eine hohe Transfektionseffizienz besitzen.

Der Grundgedanke der Erfindung liegt darin, die DNA/DNA-Träger (Transfektantien)-Komplexe mit einer weiteren, stabilisierenden Polyelektrolytschicht zu umhüllen, so daß eine Aggregation in physiologischen Medien unterbunden wird, d.h. die Partikel stabilisiert werden. Die Erfindung stützt sich auf eigene Ergebnisse eines Teils der Erfinder bei der Herstellung von Nanopartikeln (Shells) und beruht auf der schrittweisen Adsorption von Schichten entgegengesetzt geladener Polyelektrolyte an die Oberfläche von Kolloidpartikeln, bzw. hier an die durch Transfektantien kondensierte DNA. Nach Aufbringen jeder Schicht ändert sich die Oberflächenladung der Partikel. Durch geeignete Polyelektrolyte können gewünschte Hülleneigenschaften, wie selektive Permeabilität, kontrollierte Freigabe, Stabilität und Biokompatibilität erreicht werden.

Ein erfindungsgemäßes Ziel ist es, anionische Polyelektrolyte zu finden, die kationische DNA-Komplexe gegen Aggregationstendenzen unter physiologischen Bedingungen stabilisieren und zu tranfektionsaktiven Komplexen mit negativer Oberflächenladung führen. Die letztere Bedingung ist erforderlich, um eine Wechselwirkung mit den negativen Serumproteinen auszuschließen, die zum schnellen Verlust der Komplexe in vivo führen würde. Die Bindung an die negative Zelloberfläche soll andererseits über Liganden-Rezeptorwechselwirkung realisiert werden.

Das erfindungsgemäße Verfahren zum Gentransfer in tierische Zellen ist dadurch gekennzeichnet, daß a) Vektor-DNA zur Herstellung von transfizierenden Komplexen mit polykationischen Substanzen, wie Peptiden, bestehend vorwiegend aus Lysinen und Argininen, Kernproteinen wie H1 oder HMG1, synthetischen Polykationen wie Polyethylenimin (PEI) und Polyaminosäuren, wie beispielsweise Polylysin (hier exemplarisch untersucht), in Wechselwirkung gebracht wird. Dies erfolgt in wäßrigem Medium, um eine Aggregation der entstehenden Komplexe zu verhindern. In einem 2. Schritt b) wird auf diese Komplexe, die i.a. eine positive Oberflächenladung aufweisen, durch Mischung mit einem Polyanion eine negative Schicht aufgetragen. Diese Schicht, die bevorzugt aus Transfer-RNA oder synthetischen Polyanionen besteht, verhindert bei Übertragung der Komplexe in physiologische Salzbedingungen deren Aggregation. Erfindungsgemäß können als Polyanionen auch synthetische Polyanionen oder biologische relevante Oligoribonukleotide eingesetzt werden. Es sei erwähnt, daß eine Reihe anderer untersuchter Polyanionen das Auftreten von Aggregaten nicht verhindert (siehe oben). Um zu belegen, daß die erhaltenen Komplexe die Kriterien der Schichtassemblierung (geringe Größe, geringe Polydispersität, negatives Zeta-Potential) tatsächlich erfüllen, werden sie in physiologischer Salzlösung bezüglich ihrer Größenverteilung durch dynamische Lichtstreuung, AFM oder Elektronenmikroskopie sowie ihres Zeta-Potentials untersucht. Die erhaltenen Partikel erfüllen diese Kriterien und erweisen sich im Transfektionsexperiment als aktiv. Wegen ihrer negativen Oberflächenladung, die eine starke Wechselwirkung mit der Zelloberfläche behindert, ist die Transfektionseffizienz geringer, als von DNA/Polylysin-Komplexen allein, die weitgehend aggregiert sind. Dies erhöht jedoch die Erwartung, durch Einführung von Liganden für Zelloberflächenrezeptoren, die Effizienz des targetierten Gentransfers verbessern zu können. Eine einfache Methode zur Herstellung ligandierter Shells ist die Addition kationischer Peptide mit Ligandensequenzen (z.B. K₁₆-cRGD), die als dritte äußere Schicht der Shells fungiert. Es kann aber auch ein anionisches Peptid mit Ligandensequenzen (z.B. E₁₆-cRGD) den Polyanionen der äußeren Schicht beigemischt werden. Ein Problem hierbei ist jedoch die Gefahr von Aggregation, der durch sorgfältige Titration der zugefügten Peptidkomponente in definierten Konzentrationsschritten vorgebeugt werden muß.

Durch die Anwesenheit von lysosomolytischen Substanzen wie z.B. Chloroquin im Postinkubationsmedium kann die Transfektionsleistung erfindungsgemäß weiter gesteigert werden. Aber auch eine lysosomolytische Komponente z.B das polykationische Polyethylenimin (PEI) kann in die Partikel integriert werden. Mit dieser Zielstellung läßt sich Polylysin durch Polyethylenimin (PEI) ersetzen. PEI/DNA-Komplexe sind befähigt, Endosomen oder Lysosomen zu verlassen. Ein großer Vorteil der Erfindung ist die Möglichkeit, eine homogene, transfektionsaktive Partikelpopulation geringer Größe zu schaffen (im vorliegenden Beispiel unter Verwendung von Polylysin von ca. 100 nm), die als ein Modell für Untersuchungen zum in vivo Gentransfer genutzt werden kann.

Erfindungsgemäß kann das dargestellte Verfahren zur Zelltransfektion von verschiedenen kultivierten Zellinien angewandt werden. Auch ein Gentransfer in Gewebe- bzw. Organkulturen ist möglich. Ein in vivo Gentransfer ist bei ausreichender Stabilität der Partikel erreichbar, erfindungswesentlich ist die Verwendung von RNA und synthetischen Polyanionen (Polyvinylsulfat, Poly-Natrium-Styrensulfonat) als Polyanionen zum Aufbau der Shells geschützt werden. Ähnliches gilt für andere biokompatible Polyanionen, die dem erfindungsgemäßen Anspruch der Aggregationsfreiheit in Salzlösung gerecht werden. Mit den erwähnten Polyanionen erhält man erstmalig unter physiologischen Salzbedingungen aggregationsfreie Partikel geringer Größe, die transfektionsaktiv sind.

Im einzelnen läuft das erfindungsgemäße Verfahren wie folgt ab:

### 1. In vitro Transfektion

a) Das Peptid K₁₆ (Oligolysin), K₁₆-cRGD (integrinspezifisches K₁₆), H1-Histon, Polylysin oder PEI wird mit Vektor-DNA in waßrigem Medium in Wechselwirkung gebracht. Für einen Transfektionsansatz mit 2 · 10⁵ Zellen werden 2 µg DNA eingesetzt.
b) Anschließend wird Transfer-RNA (hier Typ V aus wheat germ von Sigma) zu den Komplexen im Überschuß (hier DNA : Polylysin : RNA = 1 :1.5 : 6) addiert und durch Addition von 5 M NaCl-Lösung in physiologische Ionenstärke überführt. Die Konzentrationsverhältnisse der einzelnen Komponenten sind wegen der Aggregationsgefahr zu optimieren. Anschließend werden die Shells zur Transfektion eingesetzt.

### 2. In vivo Gentransfer

Erfindungswesentlich ist die neue in vivo Applikationsvariante, die durch die Patentansprüche geschützt werden soll: Shells werden in Pluronic F127-Gel suspendiert. Dies ist durch Mischen mit dem Gel bei 4°C möglich, da bei dieser Temperatur das Gel in flüssiger Form vorliegt. Auf das freipräparierte Organ, Haut oder Tumor wird das beladene Gel als Schicht aufgetragen, das bei 37° C fest wird. Die Operationswunde wird verschlossen. Innerhalb einer Inkubationszeit von bis zu 3 Tagen löst sich das Gel auf, die Shells werden von den Geweben aufgenommen und das Fremdgen kommt zur Expression, die nach insgesamt 7 Tagen untersucht wird. Auch andere Applikationsweisen, wie die direkte Injektion in Tumoren, waren erfolgreich.

Anschließend wird die Erfindung an Beispielen erläutert, auf die sie aber nicht beschränkt sein soll.

### Ausführungsbeispiele

### 1. Herstellung und Charakterisierung von DNA-Polyelektrolyt-Shells für die in vitro Transfektion von Zellkulturen

Vektor-DNA und kationisches Polymer werden wie oben beschrieben bei Zimmertemperatur und im Überschuß des Polykation in Wasser gemischt. Hier wird pCMV Luc und Polylysin des M_{w} 58 000 im Verhältnis 1 : 1.5 (w/w) auf der Basis von 2 µg DNA verwendet. Zu diesen Komplexen wird im Überschuß Transfer-RNA (Typ V oder XI von Sigma) zugegeben, so daß ein Mischungsverhältnis DNA : Polylysin : RNA von 1 : 1.5 : 6 (g/g/g) entsteht. Durch Zugabe von 3 M NaCl wird die Lösung auf physiologische Salzkonzentration gebracht. Dieser Ansatz wird für Transfektionszwecke direkt verwendet.

Ähnliche Formulierungen wurden mit Polyethylenimin (PEI) als Polykation und Polyvinysulfat (PVS) oder Poly-Natrium-Styrensulfonat (PNSS) als Polyanionen hergestellt.

Zur physikochemischen Analyse werden bis zu 10 derartige Ansätze vereinigt. Die entstandenen Shells werden durch dynamische Lichtstreuung im Zetasizer 3000 HS (Malvern Instr. Ltd, Malvern, England) auf ihre Größe und Aggregationsneigung untersucht.

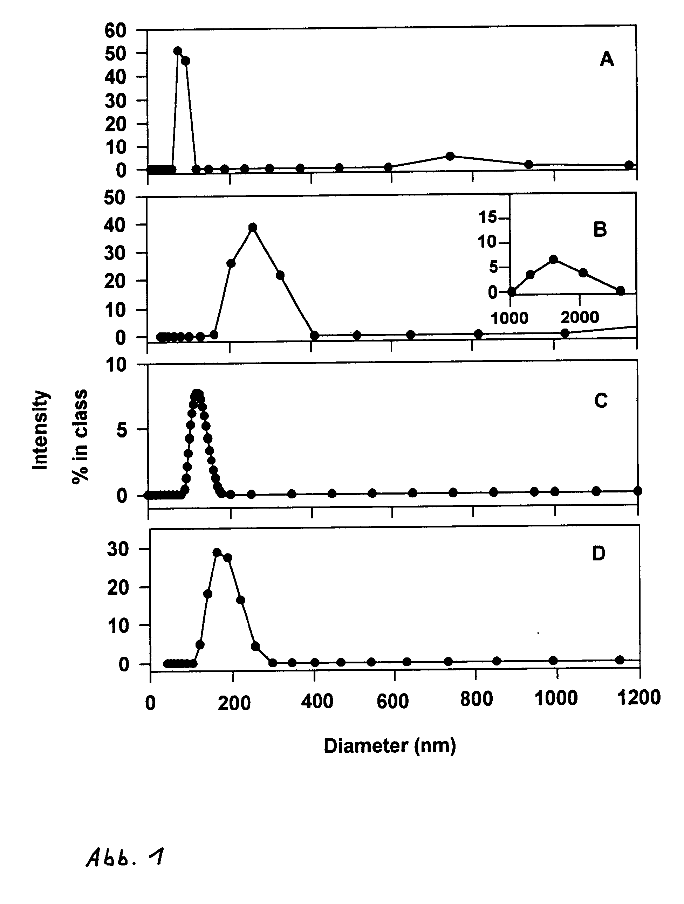

Abb. 1 zeigt die Komplexentstehung ausgehend vom Polylysin/DNA-Komplex in Wasser. Die schrittweise Veränderung dieser Komplexe nach Addition der DNA und Überführung in NaCl werden dargestellt. Es bildet sich auf der Basis von Ladungsumverteilungen eine relativ homogene Verteilung der Komplexgrößen aus.

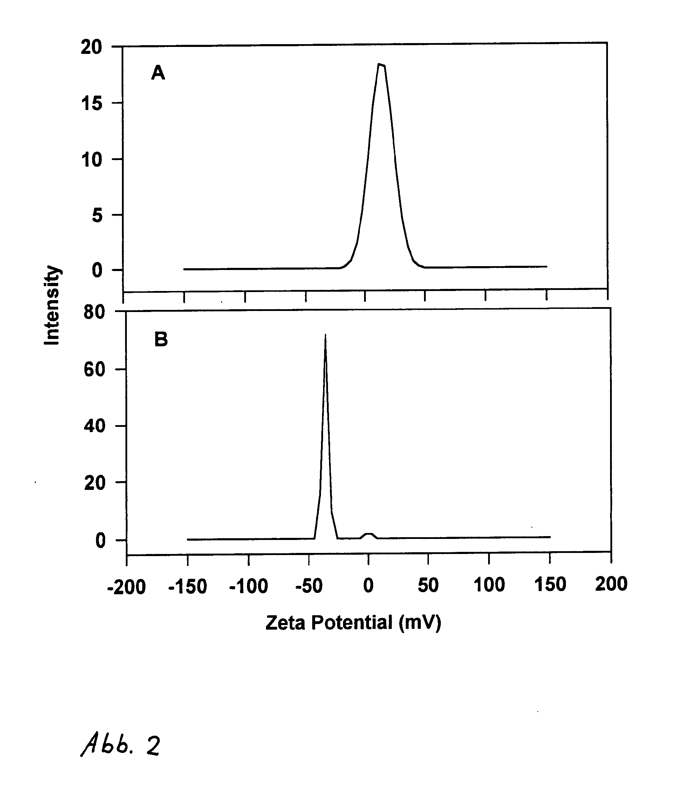

Abb. 2 zeigt das Zeta-Potential der Shells (DNA : Polylysin : RNA, 1 :1.5 : 6 w/w/w), das im gleichen Gerät gemessen wurde, im Vergleich zum Polylysin/DNA (1 : 1,5 w/w)-Komplex, der den Shells zugrunde liegt. Es ist erkennbar, daß der positive Polylysin/DNA-Komplex (+ 21.2 mV) wie erwartet in das negativ geladene Shell (- 37.1 mV) übergeht.

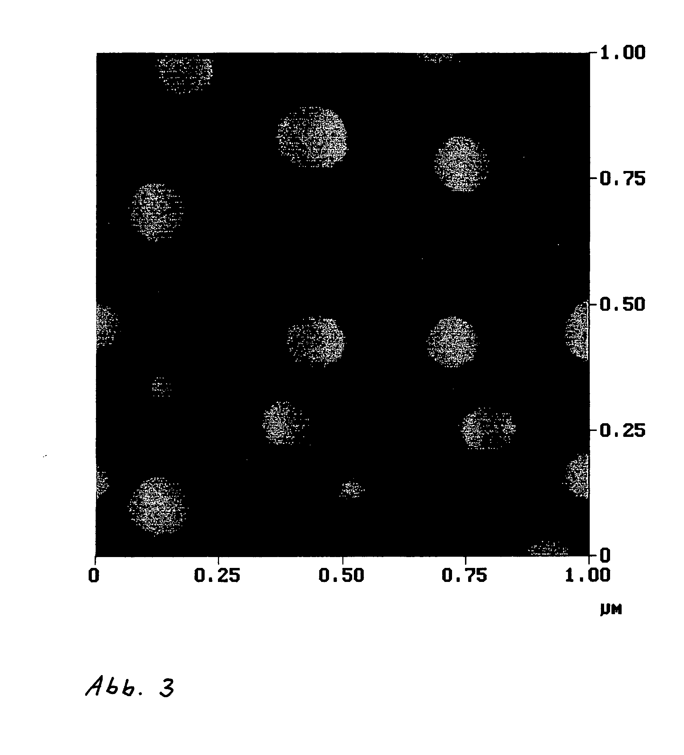

Im Gegensatz zu den Polylysin/DNA-Komplexen können diese Shells nicht oder nur teilweise bei 4000 x g für 2 min abzentrifugiert werden. Dieser einfache Test, der eine reduzierte Aggregationsneigung demonstriert, kann zu einer ersten Identifizierung der Partikel herangezogen werden. Abb. 3 zeigt derartige Shells, die durch Rasterkraftmikroskopie abgebildet wurden.

### 2. In vitro Transfektion von ECV 304-Zellen mit DNA-Polyelektrolyt-Shells

Die wie unter 1. hergestellten fertigen Shells auf der Basis von 2 µg DNA (pCMV Luc) in 200 µl Arbeitspuffer (0.15 M NaCl, 10 mM Tris-HCl, pH 7.6) werden mit 0.8 ml Zellkulturmedium aufgefüllt und zu den gewaschenen Zellen gegeben (hier 2 · 10⁵ Zellen). RPMI wird als Zellkulturmedium eingesetzt. Die Transfektionsmischung bleibt 2-4 Std. auf den Zellen, die Zellen werden mit RPMI gewaschen, frisches Kulturmedium (RPMI, 10 % FKS), gegebenenfalls mit 0.1 mM Chloroquin (oder 2 mM CaCl₂) versetzt, als Posttransfektionsmedium hinzugefügt und ca. 24 Std in einer 5 % CO₂- Atmosphäre inkubiert. Anschließend wird mit dem Assay bzw. bei stabiler Expression mit der Selektion begonnen. Beim Posttransfektionsansatz verbleibt Chloroquin für 24 Std. auf den Zellen. In diesem Fall ist die Toxizität des Ansatzes erheblich geringer, als wenn die DNA für die ganze Zeit anwesend wäre. Die Ergebnisse sind in Abb. 3 dargestellt. Nach Abzentrifugieren der Shells bei 4000 x g und 2 min ist die Transfektionseffizienz des Überstands zwar geringer, als ohne Zentrifugation, aber sie ist nicht Null, wie es bei Polylysin allein der Fall ist (der Leerwert liegt bei 2000 RLU) . Dies bedeutet, daß ein Teil der Partikel nicht abzentrifugiert werden kann, also von geringer Größe ist. Die RLU-Werte bedeuten RLU/20 µl Zellysat von 150 µl/Well. Um die RLU-Werte auf mg Protein zu beziehen, hätte man sie mit dem Faktor 100 zu multiplizieren. Mit diesem Typ von Shells werden demnach 10⁷ RLU/mg Protein erreicht.

Mit Shells vom Typ DNA(pCMV Luc)/PEI/PVS im Verhältnis 1 : 1 : 6 (g/g/g) und DNA/PEI/PNSS im Verhältnis 1 : 1 : 1 werden Transfektionsraten von 10⁷-10⁸ RLU/mg Protein auf ECV 304-Zellen erzielt.

### 3. In vivo Gentransfer in die Carotis-Arterie der Ratte

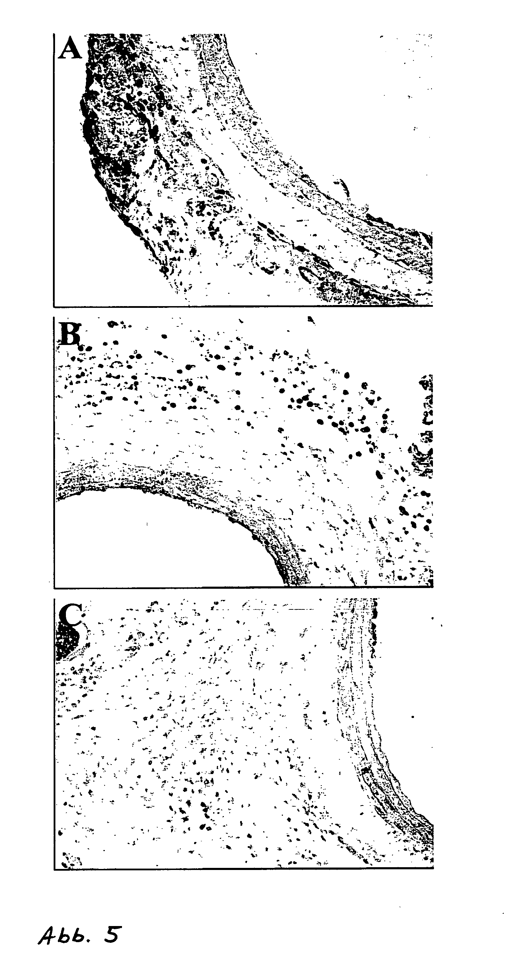

Es wurden Shells auf der Basis von DNA/PEI/RNA (pcDNA3 βGal/PEI/tRNA) im Massenverhältnis 1 :1 :1 oder DNA/PEI/PVS im Verhältnis 1 :1 :6 in Wasser hergestellt. Zum Einsatz gelangten 3,3 µg DNA bei den RNA-Shells und 6,6 µg bei den PVS-Shells. Zu den zuerst durch Mischen von gleichen Volumina PEI und DNA hergestellten PEI/DNA-Komplexen wurden die Anionen tRNA oder PVS zugegeben. Das Gesamtvolumen betrug 0.1 ml. Die Shells wurden elektronenmikroskopisch analysiert. Sie lagen bevorzugt als monomere Einheiten (< 100 nm) mit wenig Aggregaten vor. Ihre Oberflächenladung war negativ, wie aus der Kontrastierung nach Uranylacetatfärbung geschlossen wurde. PluronicF127-Gel wurde angesetzt (0,4 ml) und bei 4° C mit den Shells vermischt (bei 4° C ist das Gel flüssig). Nach Stehenlassen des Gels auf Eis für 10 min wird 3 M NaCl- Lösung bis zu einer Endkonzentration von 0.15 M zum Gel addiert. Die Carotisaorta von Ratten wurde nach Öffnung des Tieres in einer Länge von ca. 1,5 cm freipräpariert. Auf ein Segment von 1 cm Länge werden 0,5 ml Gel aufgeschichtet und die Operationswunde nach Festwerden des Gels bei 37 °C verschlossen. Die Eigenschaft des Pluronic-Gels, bei 37 °C auszuhärten, ermöglicht erst diese Art der Applikation. Innerhalb von 3 Tagen Inkubationszeit löst sich das Gel auf, wobei die Shells in direkte Wechselwirkung mit der Aorta treten und von dieser aufgenommen werden. Nach Entnahme des Aortasegmentes nach insgesamt 7 Tagen wird dieses fixiert, der Galaktosidasefärbung unterworfen, im tiefgefrorenen Zustand mit einem Mikrotom in Scheibchen von 8 µm Dicke geschnitten und auf Objektträger gebracht. Die mikroskopische Beobachtung erbrachte das Ergebnis, daß β-Gal in > 50% der Zellen der Periadventitia und Adventitia und wenigen Zellen der Media exprimiert war (Fig. 5). Die Expression der Reporter-DNA konnte abnehmend bis zu einer Tiefe von ca. 15 Zellschichten nachgewiesen werden. Shells sind also in der Lage, tief in Gewebe einzudringen. Kontrollversuche mit PEI/DNA-Komplexe zeigten eine deutlich geringere β-Gal- Expression.

### 4. Direkte Injektion von Shells in Tumore

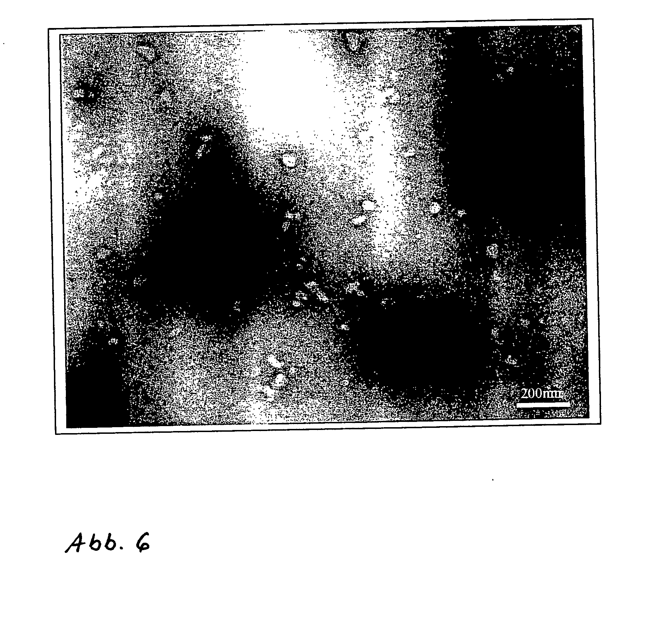

Transplantationstumore in Nude-Mäusen wurden mit humanen Tumorzellen, die vorher auf ihre Transfektionseffizienz mit Shells in vitro untersucht wurden, erzeugt. Die besten in vitro Transfektionsraten wurden im Bereich von 10⁸ RLU/mg Protein bei HCT-116 (Colon)- und MCF-7 (Mamma)-Zellen beobachtet. Es wurde mit HCT-116 erzeugten Tumoren gearbeitet. Nach ca. 10 Tagen haben die Tumoren einen Durchmesser von ca. 4 mm. Es wurden DNA/PEI/PVS-Shells und DNA/PEI/PNSS-Shells mit einem Massenverhältnis von (1 :1 :6) bzw. (1 : 1 : 11) auf der Basis von nur 1.65 µg bzw. 3.5 µg DNA (pCMV Luc) hergestellt (siehe Anwendungsbeispiele 1.). Bei den PVS-Shells befanden sich DNA und PEI vor dem Mischen in etwa gleichem Volumen (12,5 µl). Bei den PNSS-Shells wurden zu 6,3 µl DNA 3,7 µl PEI addiert. Nach dem Mischen wurden die PEI/DNA-Komplexe 10 min stehengelassen. Anschließend wurde PVS bzw. PNSS so addiert, daß sich beide Typen von Shells nach Mischen der Komponenten in 50 µl 20 mM HEPES befanden. Dann wurden 8,8 µl einer 1 M NaCl zugegeben, so daß die Endkonzentration 0,15 M NaCl betrug. Die Shells wurden entweder nach 15 min elektronenmikroskopisch mit ähnlichem Ergebnis wie unter 3. charakterisiert (Abb. 6) oder durch die Haut direkt in die Tumoren injiziert. Nach 24 Std. wurden die Tiere getötet, die Tumoren entnommen und das Homogenat mittels eines Luciferase-Reportergenassays und eines Luminometers auf Luciferase-Aktivität analysiert.

Die Ergebnisse waren reproduzierbar und erbrachten hohe Effizienzen: (8,2 ± 4,15 s.d.) 10⁵ RLU/Tumor für die PNSS-Shells und (2,3 ± 1,5 s.d.)10⁵ RLU/Tumor für die PVS-Shells. Die PEI/DNA-Kontrollen (1:1.5 g/g) waren positiv (5, 5 ± 6,2 s.d.) 10⁵ RLU/Tumor. Bei der Bewertung dieses Ergebnisses ist jedoch zu berücksichtigen, daß es sich bei den Shells um Partikel mit negativer Oberflächenladung handelt, während PEI/DNA Komplexe positiv geladen sind. Dies deutet auf verschiedene Mechanismen und möglicherweise auch auf verschiedene Zelltypen im Tumor hin, die transfiziert wurden. Weiterhin ist positiv hervorzuheben, daß bei Shells unspezifische Wechselwirkungen mit negativ geladenen Serumkomponenten und Komponenten der extrazellulären Matrix ausgeschlossen werden können.

### Abbildungslegenden

Abb. 1 Herstellungskontrolle von DNA-Polyelektrolyt-Shells anhand der Größenbestimmung mittels quasielastischer Lichtstreuung. Es sind die Streuintensitäten als Funktion der Partikelgröße (Größenverteilung) dargestellt.
   A: DNA/-Polylysin-Komplexe (1 : 1,5 w/w) in Wasser, B: DNA/Polylysin-Komplexe (1 : 1,5 w/w) in 0.15 M NaCl. Das Insert zeigt Aggregate mit > 1000 nm. C: DNA/Polylysin/tRNA- Shells (1 : 1.5 : 6 w/w/w) in Wasser, zentrifugiert mit 4000 x g für 4 min. als Überstand. D: DNA/Polylysin/RNA-Shells (1 : 1.5 : 6 w/w/w) in Wasser, zentrifugiert mit 4000 x g für 2 min (Überstand), danach Überführung in 0,15 M NaCl. Die vermessenen Ansätze beziehen sich auf 10 µg DNA.
Abb. 2 Zeta-Potential von DNA/Polylysin-Komplexen (1 : 1,5 w/w) in Wasser (A) und DNA/Polylysin/ tRNA-Shells (1 : 1,5 : 6 w/w/w) in Wasser (B).
Abb. 3 Rasterkraftmikroskopie von DNA/Polylysin/tRNA-Shells (1 : 1,5 : 6 w/w/w) in TAE-Puffer (40 mM Tris-Acetat, 1 mM EDTA).

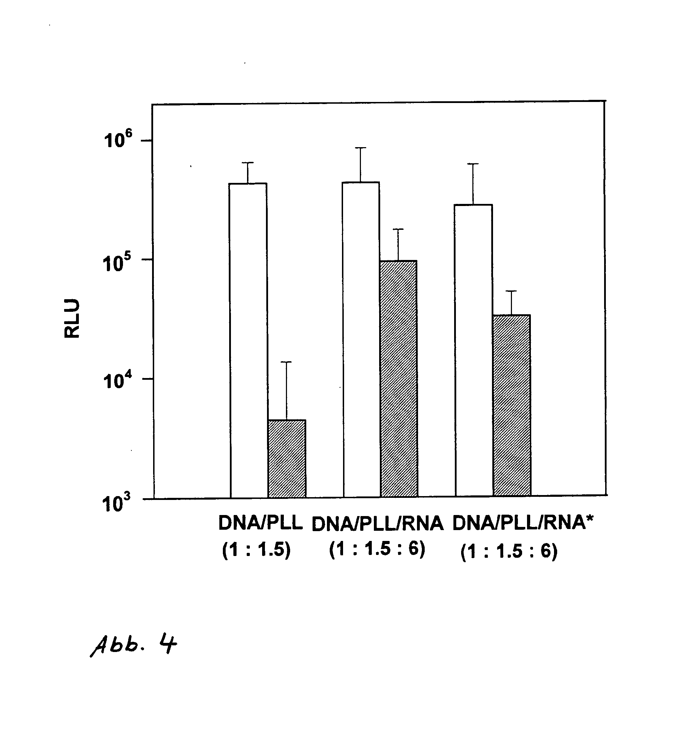

Abb. 4 Transfektion von ECV 304- Zellen mit DNA/Polylysin-Komplexen (1 : 1.5 w/w) und DNA/Polylysin/tRNA-Komplexen (1 : 1,5 : 6 w/w/w), hergestellt mit 2 handelsüblichen Transfer-RNAs von Sigma. Die Lösung der Shells (0,2 ml) enthielt 0.15 M NaCl. Die Shells wurden in zentrifugierter Form als Überstand (schraffiert) oder ohne Zentrifugation (weiß) zu 0,8 ml Transfektionsmedium (RPMI) addiert und zu den Zellen gegeben . 2 µg pCMV Luc DNA, 2 x 10⁵ ECV 304-Zellen, 2 Std. Transfektion in RPMI, Postinkubation in RPMI, 0.1 mM Chloroquine.
Abb. 5 Expression von β-Galactosidase in der Carotis-Arterie (Ratte) nach in vivo Gentransfer von Shells, die in Pluronic-Gel F127 nach Freilegung der Arterie appliziert wurden. (A) DNA/PEI/tRNA (1 : 1 : 1 w/w/w), (B) DNA/PEI/PVS (1 : 1 : 6 w/w/w), (C) Kontolle DNA/PEI (1 : 1 w/w). r bedeutet das Verhältnis Polyanion/DNA (w/w). Es wurden 3,3 µg pcDNA β-Gal bei RNA- Shells oder 6,6 µg DNA bei PVS-Shells/ 0,1 ml Gesamtvolumen eingesetzt und mit 0,4 ml PluronicF127 bei 4° C vermischt und auf die Arterie geschichtet. Der β-Galactosidase-Nachweis erfolgte nach 7 Tagen.
Abb. 6 Electronenmikroskopie von DNA/PEI/PNSS-Shells (1 : 1 : 11 w/w/w), wie sie zur direkten Tumorinjektion verwendet wurden. 3,5 µl pCMV Luc wurden als Shells in 50 µl Gesamtvolumen unter physiologischen Salzbedingungen angesetzt. Zur Injektion. gelangten ca. 50 µl Shells/Tier. Kontrastierung mit Uranylacetat.

## Patentansprüche

1. Verfahren zum Gentransfer in tierische Zellen **gekennzeichnet dadurch, daß** Vektor-DNA in wäßriger Lösung durch eine aufeinanderfolgende Addition entgegengesetzt geladener Polyelektrolyte in kompakte Nanopartikel verpackt und nachfolgend die Transfektion der Zellen durchgeführt wird.

2. Verfahren zum Gentransfer in tierische Zellen nach Anspruch 1 **gekennzeichnet dadurch, daß** die Shells in physiologische Salzlösung überführt werden, in der die Transfektion durchgeführt wird.

3. Verfahren zum Gentransfer in tierische Zellen nach Ansprüchen 1 und 2 **gekennzeichnet dadurch, daß** die Vektor-DNA
- im ersten Schritt mit Polykationen in Wechselwirkung gebracht und kondensiert wird,
- im zweiten Schritt mit Polyanionen gemischt und eine Schicht aufgetragen wird
- und diese Schritte ggf. wiederholt werden.

4. Verfahren zum Gentransfer in tierische Zellen nach Ansprüchen 1 und 2 **gekennzeichnet dadurch, daß** als Polykationen synthetische Polykationen, Peptide, Kernproteine oder Polyaminosäuren eingesetzt werden.

5. Verfahren zum Gentransfer in tierische Zellen nach Ansprüchen 1 und 2 **gekennzeichnet dadurch, daß** als synthetische Polykationen Polyethylenimin (PEI) eingesetzt wird.

6. Verfahren zum Gentransfer in tierische Zellen nach Ansprüchen 1 und 2 **gekennzeichnet dadurch, daß** als Polykationen Peptide aus vorwiegend Lysin und/oder Arginin eingesetzt werden.

7. Verfahren zum Gentransfer in tierische Zellen nach Ansprüchen 1 und 2 **gekennzeichnet dadurch, daß** als Polykationen die Kernproteine H1-Histon oder HMG1 eingesetzt werden.

8. Verfahren zum Gentransfer in tierische Zellen nach Ansprüchen 1 und 2 **gekennzeichnet dadurch, daß** als Polykationen Polylysin, Oligolysin K₁₆ oder K₁₆-Fusionspeptide eingesetzt werden.

9. Verfahren zum Gentransfer in tierische Zellen nach Ansprüchen 1 und 2 **gekennzeichnet dadurch, daß** als Polyanionen Transfer-RNA, DNA oder Oligoribonukleotide eingesetzt werden.

10. Verfahren zum Gentransfer in tierische Zellen nach Ansprüchen 1 und 2 **gekennzeichnet dadurch, daß** als Polyanionen Antisenseribonukleotide oder Mischungen aus Antisenseribonukleotiden und Transfer-RNA oder andere Polyanionen eingesetzt werden.

11. Verfahren zum Gentransfer in tierische Zellen nach Ansprüchen 1 und **gekennzeichnet dadurch, daß** als Polyanionen biokompatible anionische Polymere eingesetzt werden, die eine Aggregation der Komplexe unter physiologischen Salzbedingungen verhindern.

12. Verfahren zum Gentransfer in tierische Zellen nach Ansprüchen 1 und 2 **gekennzeichnet dadurch, daß** als Polyanion Polyvinysulfat eingesetzt wird.

13. Verfahren zum Gentransfer in tierische Zellen nach Ansprüchen 1 und 2 **gekennzeichnet dadurch , daß** als Polyanion Poly-Natrium-Styrensulfonat eingesetzt wird.

14. Verfahren zum Gentransfer in tierische Zellen nach Ansprüchen 1 und 2 **gekennzeichnet dadurch, daß** als Polykation Polylysin und als Polyanion Transfer-RNA eingesetzt wird.

15. Verfahren zum Gentransfer in tierische Zellen nach Ansprüchen 1 und 2 **gekennzeichnet dadurch, daß** als Polykation Polyethylenimin und als Polyanion Polyvinysulfat eingesetzt wird.

16. Verfahren zum Gentransfer in tierische Zellen nach Ansprüchen 1 und 2 **gekennzeichnet dadurch, daß** als Polykation Polyethylenimin und als Polyanion Poly-Natrium-Styrensulfonat eingesetzt wird.

17. Verfahren zum Gentransfer in tierische Zellen nach Ansprüchen 1 und 2 **gekennzeichnet dadurch, daß** die Vektor-DNA mit mehr als 2 Polyelektrolytschichten umhüllt wird.

18. Verfahren zum Gentransfer in tierische Zellen nach Ansprüchen 1 und 2 **gekennzeichnet dadurch, daß** folgende Parameter unabhängig voneinander gewählt werden:
- die Partikeloberflächenladung durch Wahl geeigneter Polyanionen/Polykationen an der Oberfläche
- die Ligandierung für die Aufnahme durch rezeptospezifische Endozytose durch elektrostatische Anbindung der Liganden vermittels kurzer polarer Aminosäuresegmente,
- lysosomolytische Aktivität der Shells durch Einbau von lysosomolytischen Polykationen.

19. Verfahren zum Gentransfer in tierische Zellen nach Ansprüchen 1 und 2 **gekennzeichnet dadurch, daß** die Polykationen/Polyanionen zusätzliche Liganden aufweisen.

20. Verfahren zum Gentransfer in tierische Zellen nach Ansprüchen 1 und 2 **gekennzeichnet dadurch, daß** Integrin-spezifische Peptide wie K₁₆- GGCRGDMFGCA oder ähnliche Peptide als äußere Polyelektrolytschicht verwendet wird.

21. Verfahren zum Gentransfer in tierische Zellen nach Ansprüchen 1 und 2 **gekennzeichnet dadurch, daß** für die chemische Ankopplung von Liganden Poly-Natrium-Styrensulfonat (PNSS) als Polyanion herangezogen wird.

22. Verfahren zum Gentransfer in tierische Zellen nach Ansprüchen 1 und 2 **gekennzeichnet dadurch, daß** zur Vermittlung der lysosomolytischen Aktivität als Polykationen Polyethylenimin oder Dendrimere verwendet werden.

23. Verfahren zum Gentransfer in tierische Zellen nach Ansprüchen 1 und 2 **gekennzeichnet dadurch**, die Transfektion in Gegenwart von Chloroquin oder CaCl₂ durchgeführt wird.

24. Verfahren zum in vivo Gentransfer **gekennzeichnet dadurch, daß** Vektor-DNA nach den Ansprüchen 1-21 in Polyelektrolytnanopartikel (Shells) verpackt und in lebende Systeme appliziert wird.

25. Verfahren zum in in vivo Gentransfer nach Anspruch 23 **gekennzeichnet dadurch, daß** Shells mit dem Pluronic-Gel F127 gemischt werden und in flüssiger Form auf die zu transfizierenden Gewebe/Organe/Tumoren nach deren Freilegung aufgetragen werden.

26. Verfahren zum in in vivo Gentransfer nach Anspruch 23 **gekennzeichnet dadurch, daß** Shells direkt in die zu transfizierenden Gewebe/Organe/Tumoren injiziert werden.

27. Verfahren zum in in vivo Gentransfer nach Anspruch 23 **gekennzeichnet dadurch, daß** Shells intravenös injiziert werden.

28. Verfahren zum in in vivo Gentransfer nach Anspruch 23 **gekennzeichnet dadurch, daß** Shells intraperitonial injiziert werden.

29. Verfahren zum in in vivo Gentransfer nach Ansprüchen 23, 24 und 25 **gekennzeichnet dadurch, daß** als Polykation Polyethylenimin (PEI) und als Polyanion RNA verwendet wird.

30. Verfahren zum in in vivo Gentransfer nach Ansprüchen 23, 24 und 25 **gekennzeichnet dadurch, daß** als Polykation PEI und als Polyanion Polyvinysulfat (PVS) verwendet wird.

31. Verfahren zum in in vivo Gentransfer nach Anspruch 23, 24 und 25 **gekennzeichnet dadurch, daß** als Polykation PEI und als Polyanion Poly-Natrium-Styrensulfonat (PNSS) verwendet wird.
